# EUROPEAN PATENT SPECIFICATION

(11) **EP 0 726 242 B1**
(45) Date of publication and mention of the grant of the patent: **19.07.2000**
(21) Application number: 96101741.5
(22) Date of filing: 07.02.1996
(51) Int. Cl.: C07C 15/073

(54) **Improved process for ethylbenzene production**
Verfahren zur Herstellung von Ethylbenzol
Procédé pour la préparation de l'éthylbenzène

(30) Priority: 08.02.1995 US 385262
(43) Date of publication of application: 14.08.1996
(73) Proprietor: FINA TECHNOLOGY, INC., Plano, Texas 75024 (US)
(72) Inventor: Butler, James, Houston, Texas 77062 (US); Waguespack, Jim, Baton Rouge, Louisiana 70817 (US); Hall, Ken, Baton Rouge, Louisiana 70810 (US)
(74) Representative: Leyder, Francis

(56) References cited:
- US-A- 4 922 053

## Description

This invention relates to a process for producing ethylbenzene by contacting suitable reactant under specified conversion conditions in the presence of appropriate catalyst. In another aspect, this invention relates to ethylbenzene production in which unwanted by-products, especially ortho-xylene, are suppressed while acceptable conversion and selectivity to desired ethylbenzene product are maintained by utilizing a distillation column in the PEB recycle stream to remove the undesirable lighter fractions from the stream prior to its introduction back into the reactor.

### BACKGROUND OF THE INVENTION

Ethylbenzene (EB) is used predominantly for the production of styrene monomer which is obtained through dehydrogenation of the EB. Presently much of the EB being produced is obtained by alkylation of benzene (Bz) with ethylene (Et) under a variety of alkylation conditions. One type of alkylation process which is conventional is to employ relatively high pressures and temperatures to obtain vapor phase reaction conditions wherein the ethylene and benzene are converted in the presence of catalytic materials. Both single and multiple catalyst bed processes are well known in the art.

For example, U.S. Patent 4,400,570 to Butler, et al., discloses a process for manufacturing ethylbenzene for use in styrene wherein ethylene and benzene are reacted with steam over a TEA-silicate catalyst. One problem in the production of EB by such method is the production of unwanted by-products which can be very detrimental because some of the by-products may be very difficult, or impossible, to separate from the desired EB product. Thus, as an example, the production of xylene in these types of processes is very undesirable since separation of xylenes from the EB product is very difficult from a processing standpoint due to the closeness of their boiling points. Xylenes remaining in ethylbenzene and more particularly ortho-xylene, end up as impurities in the final product, styrene, and therefore are highly undesirable elements in the EB product stream.

In a later U.S. Patent, 4,922,053 to Waguespack et al., xylene production was reduced by directing a portion of the PEB overhead recycle stream as a quench to the middle section of the reactor. Conversion rates were also improved in this process. The Waguespack '053 patent and the aforementioned patent, U.S. 4,400,570, are hereby incorporated by reference in their entirety into this application.

In addition to the requirement that the catalyst employed in such processes be selective to the desired EB product it is also desirable to obtain acceptable conversion of the reactants to alkylated products. The ability of different catalyst materials to convert the raw feed materials into products is sometimes referred to as its "activity". "Conversion" is normally measured as a percentage of the amount of feed materials which will be converted into products during the reaction. The ability of the catalyst to maintain high conversion rates (i.e., reaction activity) is very important.

Deactivation of catalysts is one major problem in catalytic alkylation processes since, even if high conversion rates are obtained initially, the failure to maintain good conversion over a long period of time requires expensive catalyst changeouts and/or regeneration procedures.

Another critical area in the formation of production of EB is in the feed/product ratio. Any factors which can significantly lower the feed/product ratios without degrading activity or conversion, and without increasing xylene production, is very desirable.

Thus, it would be desirable to obtain a process in which conversion of reactants to EB can be obtained without production of unwanted xylene by-products and without the necessity of frequently regenerating or replacing the catalytic material employed.

### SUMMARY OF THE INVENTION

It has now been discovered that alkylation of benzene with ethylene under vapor phrase reaction conditions can be effected in a process having high conversion rates and low rates of deactivation with excellent selectivity to EB and a reduced amount of xylene formation by passing the Polyethyl Benzene Overhead (PEB/OH) recycle stream through a distillation column prior to injecting the recycle into the alkylation reactor. More specifically, it has been discovered that by removing and discarding the lighter fractions of the PEB/OH recycle stream, including ortho-xylene, before reintroducing the recycle back into the reactor, excellent conversion to EB is achieved and a substantial reduction in the amount of xylenes is obtained.

Thus, in general, the present invention provides a method for producing ethylbenzene by reacting benzene and an ethylating agent in the presence of appropriate catalysts in a multizone reactor and removing undesirable portions of the PEB/OH recycle stream in a separate distillation column prior to introducing the recycle back into the reactor. Generally, temperatures in the range of from 370°C to 470°C are employed with benzene to ethylene molar ratios in the range of from 2:1 to 20:1; pressures in the range of from atmospheric to 25 atmospheres; and benzene WHSV's in the range of from 20 to 200. An appropriate catalyst such as a zeolite material like silicalite or Mobil ZSM-5 (manufactured by Mobil Oil Corporation, New York, New York) is used in the alkylation reactor. These catalysts have been discovered to achieve moderate xylene suppression and acceptable conversion and activity rates.

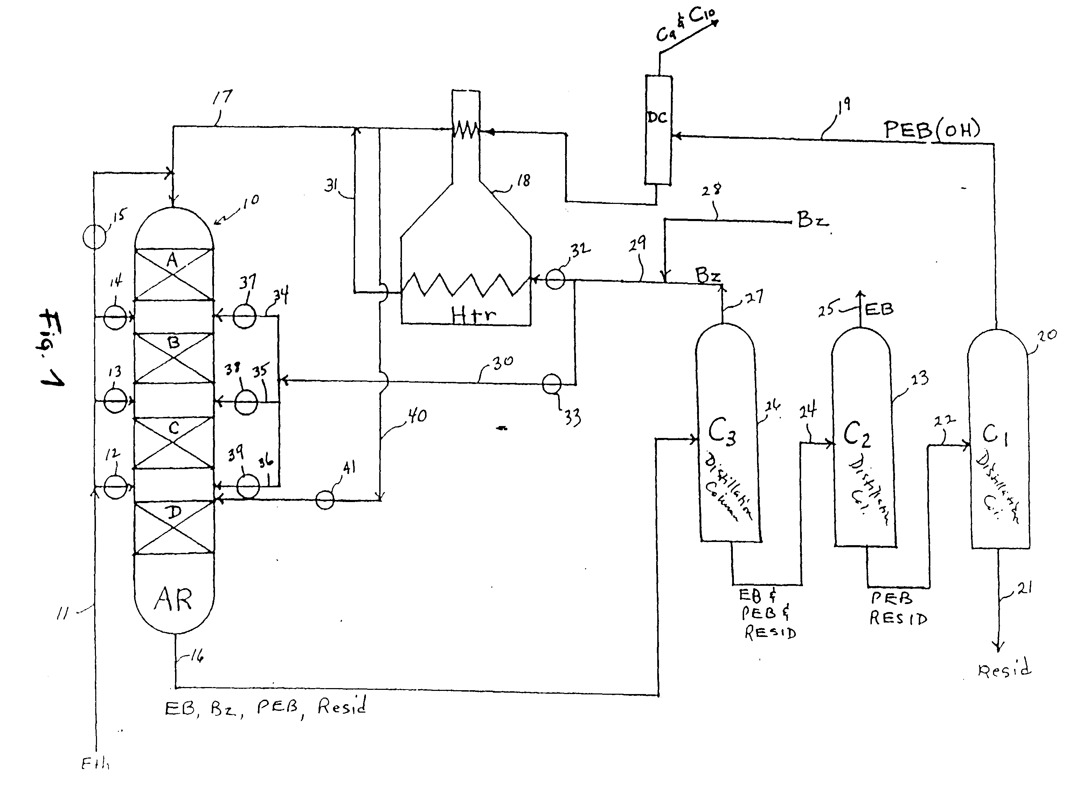

### BRIEF DESCRIPTION OF THE DRAWING

The figure illustrates schematically the alkylation process for manufacturing ethylbenzene from benzene and ethylene.

### DESCRIPTION OF THE PREFERRED EMBODIMENTS

The process of the subject invention generally comprises the steps of feeding ethylene and benzene to multiple alkylation reaction zones where the reactants are brought into contact with catalyst materials are preferably fairly stable and highly selective to the production of ethylbenzene and diethylbenzene in a temperature range of from 370°C (700°F) to 470°C (880°F). The PEB/OH reclycle stream is further distilled and separated into a light fraction and a heavy fraction. The light fraction will contain C9 and C10 hydrocarbons such as styrene, ortho-xylene, n-propylbenzene and butylbenzene. The heavy fraction will contain heavies such as diethylbenzene (DEB) and triethylbenzene (TEB) which, when contacted with the proper molar ratios of benzene, react to form the desirable product, ethylbenzene. The light fraction hydrocarbons are discarded or used for fuel value and the heavies are recycled into the main reactor to obtain more product. Additionally, xylene contamination can be reduced dramatically and regeneration of the catalyst delayed significantly.

Referring now to the schematic drawing of the EB conversion process, a 4-bed alkylation reactor (AR), designated at 10, comprises four catalytic reaction beds A, B, C and D. An ethylene (Et) feed line 11 provides a fresh supply to ethylene feedstock to reactor 10. Control valves 12-15 allow control of the ethylene feed to various sections of the reactor 10. Benzene, EB, PEB and resid formed in the reactor are withdrawn from the bottom through line 16.

Benzene is supplied to various sections of reactor 10 to react with the ethylene and form the products leaving via line 16. Two sources of benzene are preferably used to supply the reactor. A fresh benzene feedstock is supplied via benzene line 28 which joins a second benzene supply line 27 leading from distillation column C3, designated at 26. The combined benzene feed moves through line 29 which then divides into a second feed line 30. Benzene passing through line 30 is controlled by valve 33 and divides into three benzene quench lines, 34-36, controlled by flow valves 37-39. Line 34-36 provide benzene quench to reactor beds B, C and D.

Benzene feed passing through valve 32 is heated in heater 18 to 450oC, passes through control valve 31 and is mixed in line 17 with the heavy fraction from distillation column DC. It then moves through line 19, through the flue stack of heater 18 and into line 17.

The products of the alkylation reactor (Bz, PEB, EB and resid) which flow through line 16 are separated in distillation column C3, with benzene going out the top through line 27 and the remainder going out the bottom via line 24 to distillation column C2 designated at 23. The final EB product is distilled off in C2 and delivered via line 25 to the products terminal or styrene monomer facility. PEB and other hydrocarbon fractions, and resid flow out the bottom of C2 via line 22 into distillation column C1 designated at 20. PEB and other hydrocarbons are distilled off and flowed through the PEB/OH line 19. This stream is then passed through distillation column DC and separated into light and heavy fractions. Resid flows out the bottom of C1 through line 21.

In a conventional EB process, all of the PEB/OH produced from column C1 would flow through line 19, the heater flue stack, and line 17 into the top of the alkylation reactor. We have discovered, however, that the PEB/OH stream contains undesirable C9 - C10 fractions such as ortho-xylene that cause a high percentage of such fractions to show up in the production outflow line of the reactor.

This higher concentration of undesirables from the reactor adds a burden in energy costs to the entire system since it must be recycled as useless products continuously through the whole system. It also reduces the efficiency of the EB production since the outflow of reactor C2 must contain as much as 10% EB to insure that substantially all of the ortho-xylene has been removed from the EB product going out the top of C2. This also results in improved times between catalyst regeneration, increased ethylene conversion, lowered feed/product ratios and reduction of o, m and p-xylenes. The improved process resulted in a slightly higher increase in raw feed costs, but this was negligible compared to the increased efficiency and product gains with the change.

The process can be carried out using a variety of process equipment, including a reactor vessel which defines multiple alkylation zones, each containing appropriate catalyst materials. The benzene and ethylene reactants can be admixed and preheated prior to introduction into the reaction zone where they contact the catalyst beds under reaction conditions further specified hereinbelow. After a controlled residence time in the reaction zone, the converted hydrocarbon charge passes out of the reactor where the ethylbenzene products are collected by cooling and other standard recovery techniques. The excess benzene existing form the reactor is normally recycled in a conventional manner.

One particular family of catalyst materials which can be advantageously employed in the process of the subject invention can be characterized as crystalline zeolite materials which are aluminosilicates comprising three dimensional networks of Si04 and A104 tetrahedra joined by the sharing of oxygen atoms. Two types of zeolite type catalysts have been found to be preferable, silicalites and Mobil's ZSM-5.

In general, alkylation zone reaction conditions for the process of the subject invention will include temperatures in the range of from 300°C to 470°C. The zeolite type catalysts are moisture-sensitive and tend to degrade in the presence of moisture (steam), therefore care is taken to prevent the presence of moisture in the reaction.

An excess of benzene to ethylene is normally employed and in general is In the range of from 2:1 to 20:1 molar ratio of
Benzene:ethylene. Since lower benzene:ethylene ratios result in higher Percentage of ethylbenzene, lower molar ratios within this range are Preferred. Weight hourly space velocities (WHSV's) of benzene employed in the process of the subject invention can be in the range of from 20 to 200 with WHSV's in the range of from 40 to 150 being preferred.

Operating pressures between atmospheric and 25 atmospheres can be Used with a range of from 10 to 15 atmospheres being preferred.

When the present invention is utilized in an existing ethylbenzene production facility by the insertion of the aforementioned distillation column DC in the PEB/OH recycle line, increased catalyst life is obtained and, additionally undesirable xylenes coming from the reactor are reduced in content by half. When the distillation column is not utilized in the PEB/OH line and PEB's are recycled into the reactor, catalyst life is greatly shortened over that without PEB recycle. However, when the distillation column DC was utilized in the PEB recycle line, catalyst life was restored to the same level that it was when no PEB recycle was fed to the reactor. The orth-xylenes in the EB product were cut by one-half and the amount of lost EB coming out of the bottom of column C2 was reduced from 10 percent to 1 percent, a recapture of 90 percent of the normally-lost EB.

Although a specific preferred embodiment of the present invention has been described in the detailed description above, the description is not intended to limit the invention to the particular forms of embodiments disclosed therein. Thus, the invention covers all changes and modifications which do not constitute departure from the scope of the invention.

## Claims

1. In a process for producing ethylbenzene wherein benzene and ethylene under alkylation reaction conditions are contacted with an appropriate catalyst in a multibed reactor (10) to form reaction products which include polyethylbenzenes and ethylbenzenes (16), distilling the polyethylbenzenes from said reaction products (C3,C2,C1), and recycling said polyethylbenzenes back to said reactor (19); the improvement comprising: passing said recycled polyethylbenzenes, prior to said reactor, through a distillation column (DC); distilling light fractions therefrom, including C9 and C10 hydrocarbons, ortho-xylenes, n-propylbenzene and butylbenzene, in said column and removing said distilled fractions from the process; and, transmitting the remaining heavy fractions, including diethylbenzenes and trietylbenzenes back to said reactor (17).

2. The process of claim 1 wherein said appropriate catalyst is a zeolite catalyst and said multibed reactor has at least three reactor beds.

## Patentansprüche

1. Verfahren zum Herstellen von Ethylbenzol, wobei Benzol und Ethylen unter Alkylierungsreaktionsbedingungen mit einem angemessenen Katalysator in einem Mehrbettreaktor (10) unter Bilden von Reaktionsprodukten in Kontakt gebracht werden, welche Polyethylbenzole und Ethylbenzole (16) umfassen, Destillieren der Polyethylbenzole von den Reaktionsprodukten (C3, C2, C1) und im Kreislauf führen der Polyethylbenzole zurück zu dem Reaktor (19), gekennzeichnet durch Durchleiten der im Kreilauf geführten Polyethylbenzole vor dem Reaktor durch eine Destillationssäule (DC), Destillieren leichter Fraktionen davon, einschließlich C9 und C10 Kohlenwasserstoffe, ortho-Xylole, n-Propylbenzol und Butylbenzol, in der Säule und Entfernen der destillierten Fraktionen aus dem Verfahren und Befördern der verbleibenden schweren Fraktionen einschließlich Diethylbenzole und Triethylbenzole zurück zu dem Reaktor (17).

2. Verfahren nach Anspruch 1, wobei der angemessene Katalysator ein Zeolithkatalysator ist, und der Mehrbettreaktor mindestens drei Reaktorbetten hat.

## Revendications

1. Dans un procédé pour produire de l'éthylbenzène dans lequel le benzène et l'éthylène sous des conditions de réaction d'alkylation sont mis en contact avec un catalyseur adéquat dans un réacteur à lits multiples (10) pour former les produits de la réaction qui comprennent des polyéthylbenzènes et éthylbenzènes (16), distiller les polyéthylbenzènes pour les séparer des produits de la réaction (C3,C2,C1), et recycler ces polyéthylbenzènes de retour dans ce réacteur (19); le perfectionnement comprenant: faire passer les polyéthylbenzènes recyclés, à l'avant de ce réacteur, au travers d'une colonne de distillation (DC); distiller les fractions légères pour les séparer, y compris les hydrocarbures C9 et C10, les orthoxylènes, n-propylbenzène et butylbenzène dans cette colonne et éliminer ces fractions distillées du procédé; et transmettre les fractions lourdes restantes, y compris les diéthylbenzènes et triéthylbenzènes de retour dans ce réacteur (17).

2. Procédé selon la revendication 1 dans lequel ce catalyseur adéquat est un catalyseur de zéolite et ce réacteur à lits multiples comprend au moins trois lits de réacteur.
